(19) Europäisches Patentamt
European Patent Office
Office européen des brevets

(11) Publication number : **0 126 524 B2**

(12) **NEW EUROPEAN PATENT SPECIFICATION**

(45) Date of publication of the new patent specification : **24.06.92 Bulletin 92/26**

(51) Int. Cl.$^5$ : **C07C 51/02,** C07C 53/02, C07C 51/41, C07C 53/06

(21) Application number : **84301772.4**

(22) Date of filing : **15.03.84**

(54) **Process for the preparation of formic acid.**

(30) Priority : **18.03.83 GB 8307611**

(43) Date of publication of application : **28.11.84 Bulletin 84/48**

(45) Publication of the grant of the patent : **14.01.87 Bulletin 87/03**

(45) Mention of the opposition decision : **24.06.92 Bulletin 92/26**

(84) Designated Contracting States : **BE DE FR GB IT NL**

(56) References cited :
**EP-B- 0 001 432**
**GB-A- 1 028 930**
**US-A- 3 620 674**

(73) Proprietor : **BP Chemicals Limited**
**Belgrave House 76 Buckingham Palace Road**
**London, SW1W 0SU (GB)**

(72) Inventor : **Anderson, Jeffrey John**
**BP Chemicals Limited Saltend**
**Hedon Hull (GB)**
Inventor : **Hamlin, John Edward**
**BP Chemicals Limited Saltend**
**Hedon Hull (GB)**

(74) Representative : **Crack, Richard David et al**
**c/o The British Petroleum Company plc**
**Patents Division Chertsey Road**
**Sunbury-on-Thames Middlesex, TW16 7LN**
**(GB)**

EP 0 126 524 B2

## Description

The invention relates to a process for the preparation of thermally decomposable base formates and also to a process for the preparation of formic acid involving decomposition of said base formates.

In GB-A-1 028 930 there is described a process for the production of addition compounds containing formic acid by reacting 1 mol of a tertiary organic base with at least 2 mols of a highly concentrated formic acid.

In EP-A-0 095 321 there is described a process for the production of a formate salt of a nitrogenous base which comprises reacting hydrogen and carbon dioxide with the nitrogenous base using a Group VIII transition metal catalyst and using an alcoholic solvent.

Formic acid can be obtained from the formate salt or addition compound by hydrolysis with an acid stronger than formic acid but this has the disadvantage that a salt of the base is produced.

It is therefore an object of the present invention to provide an improved process for the preparation of formic acid from the aforesaid salts or addition compounds.

According to the present invention is provided A process for the preparation of formic acid from a first base formate which is either a salt or an addition compound of formic acid combined with a first base which is a $C_1$ to $C_{10}$ trialkylamine characterised in that the process comprises the steps of:

(1) contacting the first base formate with a second base of general formula:

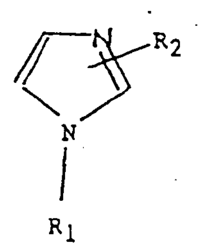

where $R_1$ is a monovalent hydrocarbon group containing 1 to 12 carbon atoms and $R_2$ is either hydrogen or $R_1$ group, which is (I) weater than the first base and (II) is less volatile than the first base hunder conditions to cause the second base to displace the first base and thereby form a second base formate which is thermally decomposable at a temperature higher than the boiling point of the first base;

(2) separating, by distillation, the first base from the second base formate, and

(3) thermally decomposing the second base formate to yield formic acid and the second base.

Conveniently the second base is a high boiling compound, for example boiling above 180°C since this facilates separation from the formic acid.

The second base has a $pK_a$ in the range 4.0 to 9.0 and is a compound of the general formula:

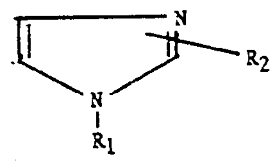 (I)

where $R_1$ is a monovalent hydrocarbon group containing 1 to 12 carbon atoms and $R_2$ is a hydrogen atom or an $R_1$ group, the total number of carbon atoms on $R_1$ and $R_2$ conveniently being not more than 20 and preferably from 4 to 12.

Suitable hydrocarbon radicals in the imidazole derivatives (I) are, in general, alkyl of 1 to 8 carbon atoms, cyclopentyl, cyclohexyl, phenyl and methyphenyl. Amongst the above, imidazole derivatives where $R_1$ is n-1-alkyl of 4 to 10 carbon atoms and $R_2$ is hydrogen or methyl are particularly suitable. Examples of such compounds are 1 - (n - 1 - butyl) - imidazole (pKa 5.9), 1 - (n - 1 - pentyl) - imidazole (pKa 5.9), 1 - (n - 1 - decyl) -imidazole (pKa 5.75), 1 - (n - 1 - butyl) - 2 - methylimidazole (pKa 7.0) and 1 - (n - 1 - pentyl) - 2 - methylimidazole (pKa 6.85).

For a definitiion of the pKa values, which are a measure of the base strengh, reference may be made, for example to Landoldt-Börnstein, 6th edition, 7th part volume II, page 900 et seq.

The first base is a $C_1$ to $C_{10}$ trialkylamine. Examples of suitable trialkylamines are trimethylamine, triethylamine, tripopylamine and tributylamine.

Depending on the identity of the first base, the second base is selected so that

(1) the second base in wealker than the first

(2) the second base formate is thermally decomposable at a temperature higher than the boiling point of the first base.

(3) the second base is less volatile than the first base.

Desirably a molar excess of the second base is employed.

The invention is illustrated by reference to the accompanying figure which is a drawing of a continuous process for operating the present invention.

A feed comprising an aqueous solution of triethylammonium formate obtained as the product from a process in which triethylamine was reacted with carbon dioxide and hydrogen in a mixture of water and alcohol as solvent and using a Group VIII transition metal catalyst as described in EP-A-0 095 321 is passed by line 2 into column 4 from which column triethylamine, alcohol and water are removed as overheads via line 6.

The triethylammonium formate in the form of a base adduct with a base:formate ratio of 1:2 to 1:3 is withdrawn from the base of column 4 via line 8 (to

which line recycled N-butyl imidazole is added as a 0.5 to 1.0 molar excess with respect to formate) and passed to column 10. In this column at 150°C and 220 mbar mercury pressure the base interchange reaction occurs, the triethylamine being displaced from the base formate and removed overhead at 39-40°C via line 12 and the formate salt of the weak base in the form of N-butyl imidazolinium formate withdrawn from the base of column 10 via line 14 and passed to column 16 where it is decomposed into anhydrous formic acid which is removed as overheads via line 18 and N-butyl imidazole which is recycled via line 20 to line 8.

The invention is illustrated by the following examples.

The distillation experiments given in Examples 1 and 2 were carried out using a 20 plate 25.4 mm Oldershaw column.

Example 1

A solution of free triethylamine (81 g), triethylammonium formate (351.5 g, formic acid:amine ratio 1:1) in water (225 g) was distilled to remove triethylamine and water. At atmospheric pressure two fractions were taken off; the first, a 10% w/w aqueous triethylamine azeotrope (18.3 g) at a head temperature of 76°C, reflux ratio 1:1, then a 25% aqueous triethylamine heterogeneous azeotrope (64.2 g) over 89-94°C head temperature, reflux ratio 2:1. At reduced pressure (220 mbar) a further two main fractions were removed; a 46% aqueous triethylamine heterogeneous azeotrope (18.6 g) at a head temperature of 37°C and water fraction (164.0 g) at 60°C, reflux ratio 1:1. This left a residue consisting of an amine formate adduct (185.5 g, formic acid:amine ratio 3:1) which contained 1% w/w water.

To 182.5 g of this residue, 600 g of N-butyl imidazole was added. The remaining triethylamine (75 g) was distilled from the mixture at 39-40°C head temperature, 220 mbar reflux ratio 1:1. Gas chromatographic analysis of the residue (690 g) showed it to contain n-butyl imidazolinium formate (384 g) in n-butyl imidazole (i.e. formic acid equivalent 110 g).

The residue was that distilled to give formic acid overhead at 26-29°C, base temperature 147°C, 47.6 mbar, reflux ratio 4:1.

Example 2

N-butyl imidazole (250 g) was added to a solution of triethylammonium formate (147 g, formic acid to amine ratio 1:1) in water (50 g). The triethylamine/water azeotrope (109 g; 10% w/w water) was distilled from the mixture at a head temperature of 77°C, reflux ratio 2:1, followed by a water fraction at 28-30°C, 46.7 mbar, reflux ratio 4:1. Finally aqueous formic acid (61% w/w acid) was taken off overhead at 30-31°C, base temperature 147°C, 46.7 mbar reflux ratio 4:1.

Example 3

N-methyl imidazole (25 g) was added to trimethylammonium formate (18 g, acid to base ratio 2.5:1). Trimethylamine (4.5 g) was distilled from the solution using a Vigreaux column (20 mm diameter) at a base temperature of 108-150°C. Gas chromatographic analysis of the residue (37.4 g) showed it to contain 25 g of N-methyl imidazolium formate (acid to base ratio 1:1) dissolved in N-methyl imidazole (11.4 g).

## Claims

1. A process for the preparation of formic acid from a first base formate which is either a salt or an addition compound of formic acid combined with a first base which is a $C_1$ to $C_{10}$ trialkylamine characterised in that the process comprises the steps of:

(1) contacting the first base formate with a second base of general formula:

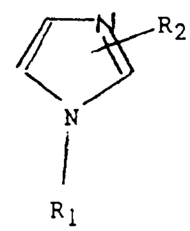

where $R_1$ is a monovalent hydrocarbon group containing 1 to 12 carbon atoms and $R_2$ is either hydrogen or $R_1$ group, which is (i) weaker then the first base and (ii) is less volatile then the first base, under conditions to cause the second base to displace the first base and thereby form a second base formate which is thermally decomposable at a temperature higher than the boiling point of the first base;

(2) separating, by distillation, the first base from the second base formate, and

(3) thermally decomposing the second base formate to yield formic acid and the second base.

2. A process as claimed in claim 1 characterised in that the second base obtained by decomposing the second base formate in step (3) is recycled to step (1).

3. A process as claimed in either claim 1 or claim 2 characterised in that the first base formate used as starting material is one obtained by reaction of carbon dioxide and hydrogen in the presence of a $C_1$ to $C_{10}$ trialkylamine and that it is first treated to at least partly remove water and, optionally some of the $C_1$ to $C_{10}$ triaklylamine, and is then contacted with the second base in step (1) and that the $C_1$ to $C_{10}$ trialkylamine is recycled to the formate salt-forming reaction stage

involving carbon dioxide and hydrogen.

4. A process as claimed in claim 1 characterised in that in the formula (I) $R_1$ is n-1-alkyl of 4 to 10 carbon atoms and $R_2$ is either hydrogen or methyl.

5. A process according to either claim 1 or claim 3 wherein the compound of formula (I) is 1-(n-1-butyl)-imidazole, 1-(n-1-pentyl)-imidazole, 1-(n-1-decyl)-imidazole, 1-(n-1-butyl)-2-methylimidazole or 1-(n-1-pentyl)-2-methylimidazole.

## Patentansprüche

1. Verfahren zur Herstellung von Ameisensäure aus einem Formiat einer ersten Base, das entweder ein Salz oder eine Additionsverbindung ist, enthaltend Ameisensäure vereinigt mit einer ersten Base, die ein $C_1$ bis $C_{10}$ Trialkylamin ist, gekennzeichnet dadurch, daß das Verfahren die Schritte von

(1) In-Kontakt-Bringen des Formiats einer ersten Base mit einer zweiten Base einer allgemeinen Formel:

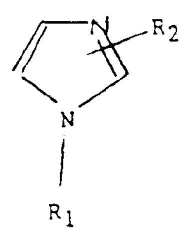

worin $R_1$ ein einwertiger Kohlenwasserstoffrest mit 1 bis 12 Kohlenstoffatomen ist und $R_2$ entweder ein Wasserstoffatom oder ein $R_1$-Rest ist, der (i) schwächer als die erste Base ist und (ii) weniger flüchtig als die erste Base ist, unter Bedingungen, damit die zweite Base die Ersetzung der ersten Base bewirkt und sich dadurch ein Formiat einer zweiten Base bildet, das thermisch bei einer Temperatur, die höher als der Siedepunkt der ersten Base ist, zersetzbar ist,

(2) Trennung durch Destillation der ersten Base von dem Formiat einer zweiten Base und

(3) thermisches Zersetzen des Formiats der zweiten Base, um Ameisensäure und die zweite Base zu erhalten, umfaßt.

2. Verfahren nach Anspruch 1, dadurch gekennzeichnet daß, die zweite Base, die durch Zersetzung des Formiats einer zweiten Base aus Schritt (3) erhalten wird, zu Schritt (1) zurückgeführt wird.

3. Verfahren nach Anspruch 1 oder 2, dadurch gekennzeichnet, daß das Formiat einer ersten Base, das als Ausgangsmaterial verwendet wird, eines ist, das durch Umsetzung von Kohlendioxid und Wasserstoff in Gegenwart von $C_1$ bis $C_{10}$ Trialkylamin erhalten wird und, daß es zuerst behandelt wird, um mindestens teilweise Wasser zu entfernen und, gegebenenfalls etwas von dem $C_1$ bis $C_{10}$ Trialkylamin, und dann mit der zweiten Base in Schritt (1) in Kontakt gebracht wird, und daß das $C_1$ bis $C_{10}$ Trialkylamin zu dem Formiat-Salz-bildenden Reaktionsschritt, an dem Kohlendioxid und Wasserstoff beteiligt sind, zurückgeführt wird.

4. Verfahren nach Anspruch 1, dadurch gekennzeichnet, daß in der Formel (I) $R_1$ ein n-1-Alkyl von 4 bis 10 Kohlenstoffatomen ist und $R_2$ entweder Wasserstoff oder Methyl ist.

5. Verfahren nach Anspruch 1 oder 3, worin die Verbindung der Formel (I) 1-(n-1-Butyl)-imidazol, 1-(n-1-Pentyl)-imidazol, 1-(n-1-Decyl)-imidazol, 1-(n-1-Butyl)-2-methylimidazol oder 1-(n-1-Pentyl)-2-methylimidazol ist.

## Revendications

1. Procédé pour la préparation de l'acide formique à partir d'un formiate d'une première base, qui est un sel ou un composé d'addition de l'acide formique combiné avec une première base, laquelle est une trialkylamine en $C_1$ a $C_{10}$, procédé caractérisé en ce qu'il comprend les étapes consistant à :

(1) mettre le formiate de la première base en contact avec une seconde base de formule générale

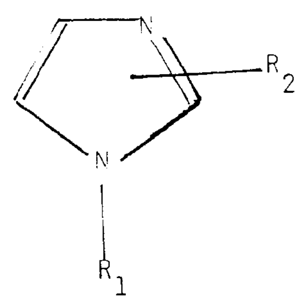

(dans laquelle $R_1$ représente un groupe hydrocarboné monovalent contenant 1 à 12 atomes de carbone, et $R_2$ représente l'hydrogène ou un groupe $R_1$, qui est (i) plus faible que la première base, et (ii) est moins volatil que la première base) dans des conditions convenant pour contraindre la seconde base à déplacer la première base et à former ainsi un formiate de seconde base, qui est thermiquement décomposable à une température supérieure au point d'ébullition de la première base ;

(2) séparer, par distillation, la première base d'avec le formiate de la seconde base ; et

(3) décomposer thermiquement le formiate de la seconde base pour obtenir l'acide formique et la seconde base.

2. Procédé selon la revendication 1, caractérisé en ce que la seconde base, obtenue par décomposition du formiate de seconde base à l'étape (3), est recyclée vers l'étape (1).

3. Procédé selon l'une des revendications 1 ou 2, caractérisé en ce que le formiate de la première base, servant de matière de départ, est un formiate obtenu par la réaction du bioxyde de carbone et de l'hydrogène en présence d'une trialkylamine en $C_1$ à $C_{10}$, et en ce qu'il est tout d'abord traité pour en enlever au moins en partie l'eau et, éventuellement, une partie de la trialkylamine en $C_1$ à $C_{10}$ puis en ce qu'il est mis en contact avec la seconde base, à l'étape (1) et en ce que la trialkylamine en $C_1$ à $C_{10}$ est recyclée vers l'étape de réaction de formation du formiate salin impliquant l'utilisation du bioxyde de carbone et de l'hydrogène.

4. Procédé selon la revendication 1, caractérisé en ce que, dans la formule (I), $R_1$ représente un groupe n-alkyle ayant 4 à 10 atomes de carbone, et $R_2$ représente un atome d'hydrogène ou un groupe méthyle.

5. Procédé selon la revendication 1 ou la revendication 3, dans lequel le composé de formule (I) est le 1-(n-1-butyl)-imidazole, le 1-(n-1-pentyl)-imidazole, le 1-(n-1-décyl)-imidazole, le 1-(n-1-butyl)-2-méthylimidazole ou le 1-(n-1-pentyl)-2-méthylimidazole.

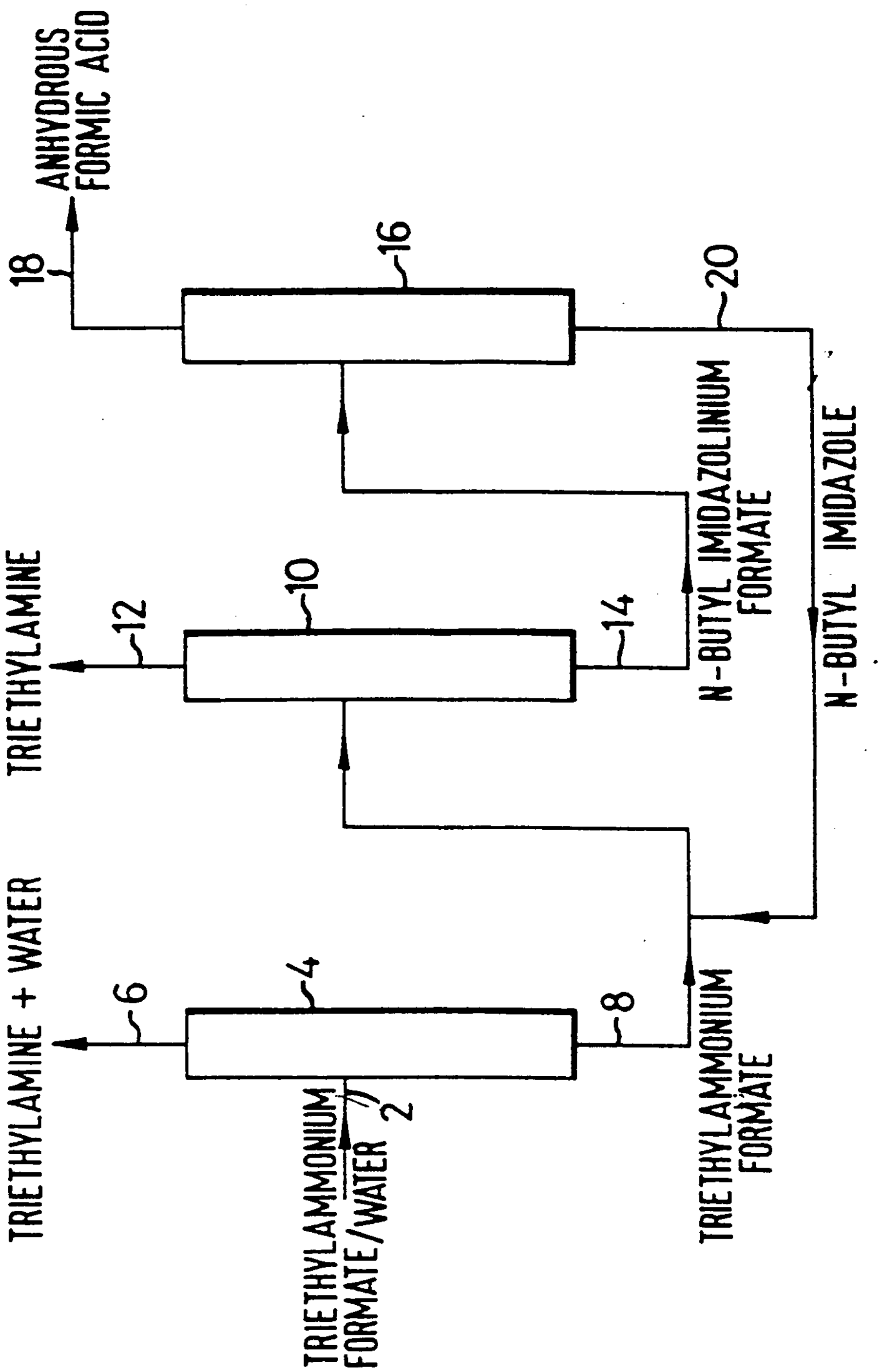
ANHYDROUS FORMIC ACID
18
16
20
N-BUTYL IMIDAZOLINIUM FORMATE
N-BUTYL IMIDAZOLE
TRIETHYLAMINE
12
10
14
TRIETHYLAMINE + WATER
6
4
8
TRIETHYLAMMONIUM FORMATE/WATER
2
TRIETHYLAMMONIUM FORMATE